# EUROPÄISCHE PATENTSCHRIFT

(11) **EP 1 028 732 B1**
(45) Veröffentlichungstag und Bekanntmachung des Hinweises auf die Patenterteilung: **11.08.2004**
(21) Anmeldenummer: 98959847.9
(22) Anmeldetag: 05.11.1998
(51) Int. Cl.: A61K 31/575, A61K 31/70, A61K 31/715, A61P 3/00

(54) **VERWENDUNG VON AUSGEWÄHLTEN PHYTOSTENOLESTERN ZUR HERSTELLUNG VON HYPOCHOLESTERINÄMISCHEN MITTELN**
USE OF SELECTED PHYTOSTENOL ESTERS FOR PRODUCING HYPOCHOLESTERAEMIC PREPARATIONS
UTILISATION D'ESTERS DE PHYTOSTENOL SELECTIONNES POUR LA PRODUCTION D'AGENTS HYPOCHOLESTERINEMIQUES

(30) Priorität: 14.11.1997 DE 19750422
(43) Veröffentlichungstag der Anmeldung: 23.08.2000
(73) Patentinhaber: Cognis Deutschland GmbH & Co. KG, 40589 Düsseldorf (DE)
(72) Erfinder: FABRY, Bernd, D-41352 Korschenbroich (DE)
(86) Internationale Anmeldenummer: PCT/EP1998/007057
(87) Internationale Veröffentlichungsnummer: WO 1999/025361

(56) Entgegenhaltungen:
- WO-A-92/19640
- WO-A-98/23277
- DE-A- 2 408 067
- FR-A- 2 228 493
- US-A- 5 244 887
- PATENT ABSTRACTS OF JAPAN vol. 096, no. 004, 30. April 1996 & JP 07 330611 A (YAKULT HONSHA CO LTD), 19. Dezember 1995

## Beschreibung

### Gebiet der Erfindung

Die Erfindung betrifft die Verwendung von Phytostenolestern, gegebenenfalls zusammen mit ausgewählten Potenzierungsmitteln zur Herstellung von Mitteln zur Verminderung des Cholesteringehaltes im Serum von Warmblütern.

### Stand der Technik

Unter hypochloesterinämischen Wirkstoffen werden Mittel verstanden, die zu einer Verminderung des Cholesteringehaltes im Serum von Warmblütern führen, ohne daß dadurch eine Hemmung oder Verringerung der Bildung von Cholesterin im Blut eintritt. Für diesen Zweck wurden bereits von Peterson et al. in **J.Nutrit. 50, 191 (1953)** Phytostenole, also pflanzliche Stenole, und deren Ester mit Fettsäuren vorgeschlagen. In die gleiche Richtung weisen auch die Patentschriften **US 3,089,939, US 3,203,862** sowie die deutsche Offenlegungsschrift **DE-OS 2035069** (Procter & Gamble). Die Wirkstoffe werden üblicherweise Brat- oder Speiseölen zugesetzt und dann über die Nahrung aufgenommen, wobei die Einsatzmengen jedoch in der Regel gering sind und üblicherweise unter 0,5 Gew.-% liegen, um zu verhindern, daß die Speiseöle eintrüben oder die Stenole bei Zusatz von Wasser ausgefällt werden. Für den Einsatz im Nahrungsmittelbereich, in Kosmetika, pharmazeutischen Zubereitungen und im Agrarsektor werden in der europäischen Patentanmeldung **EP-A1 0289636** (Ashai) lagerstabile Emulsionen der Stenolester in Zucker- oder Polyglycerinestern vorgeschlagen. Die Einarbeitung von Sitostanolestern zur Verminderung des Blutcholesteringehaltes in Margarine, Butter, Mayonnaise, Salatsaucen und dergleichen wird in der Europäischen Patentschrift **EP-B1 0594612** (Raision) vorgeschlagen. In der Internationalen Patentanmeldung **WO 98/23277** (Henkel) werden Kombinationen von Phytostenolen und/oder Phytostenolestern mit Potenzierungsmitteln zur Herstellung von hypocholesterinämischen Mitteln offenbart. Phytostenolester wie das γ-Oryzanol und fünf ähnliche Verbindungen vermindern über die Hemmung der Phospholipase A₂ - Aktivität die Aufnahme von Cholesterol über den Darm, so beschrieben in der japanischen Offenlegungsschrift **JP 07 330611** (Yakult Honsha Co Ltd).

Von Nachteil ist jedoch, daß die Phytostenolester den Nahrungsmitteln üblicherweise nur in geringen Mengen zugesetzt werden können, da ansonsten die Gefahr besteht, daß sie den Geschmack und/oder die Konsistenz der Mittel beeinträchtigen. Zur nachhaltigen Beeinflussung des Cholesteringehaltes im Blut wäre jedoch die Aufnahme größerer Mengen Phytostenolester wünschenswert. Weiterhin verbesserungswürdig ist die Geschwindigkeit, mit der die Stoffe den Gehalt an Cholesterin im Serum vermindern. Die Aufgabe der Erfindung hat folglich darin bestanden, diesen Mängeln abzuhelfen.

### Beschreibung der Erfindung

Gegenstand der Erfindung ist die Verwendung von Estern von Phytostenolen mit konjugierter Linolsäure und/oder konjugierter Fischfettsäure, gegebenenfalls zusammen mit Potenzierungsmitteln ausgewählt aus der Gruppe, die gebildet wird von Tocopherolen, Chitosanen, Phytostenolsulfaten und/oder (Desoxy)Ribonucleinsäuren, zur Herstellung von hypocholesterinämi-schen Mitteln.

Überraschenderweise wurde gefunden, daß Phytostenolester auf Basis konjugierter Linolsäure und/oder konjugierter Fischfettsäure bei der Senkung des Cholesteringehaltes im Blut eine deutlich höhere Aktivität zeigen als vergleichbare Phytostenolester, die sich von gesättigten Fettsäuren, einfach ungesättigten Fettsäuren oder mehrfach ungesättigten Fettsäuren mit zwei und mehr, nichtkonjugierten Doppelbindungen ableiten. Durch Kombination der erfindungsgemäß zu verwendenden Phytostenolester (Komponente a) mit Potenzierungsmitteln (Komponente b) aus der Gruppe der Chitosane, Phytostenolsulfate und/oder Desoxy- bzw. Ribonucleinsäuren, die selbst über keine oder nur sehr geringe hypochloesterinämischen Eigenschaf-ten verfügen, kann die Abnahme des Cholesteringehaltes im Serum weiter beschleunigt werden. In Gelatine verkapselt lassen sich sowohl die Phytostenolester als auch die Wirkstoffgemische zudem problemlos oral einnehmen.

### Phytostenolester

Unter Phytostenolen (oder synonym Phytosterolen) sind pflanzliche Steroide zu verstehen, die nur am C-3 eine Hydroxylgruppe, sonst aber keine funktionellen Gruppen tragen. In der Regel besitzen die Phytostenole 27 bis 30 Kohlenstoffatome und eine Doppelbindung in 5/6, gegebenenfalls 7/8, 8/9 oder anderen Positionen. Durch Härtung können aus den ungesättigten Stenolen die entsprechenden gesättigten Stanole erhalten werden, die von der Erfindung mitumfaßt werden. Durch Veresterung der Stenole bzw. Stanole mit ungesättigten Fettsäuren mit konjugierten Doppelbindungen, nämlich konjugierter Linolsäure (CLA) oder konjugierten Fischfettsäuren, werden die Stoffe erhalten, die die Komponente (a) bilden. Die Phytostenolkomponente der Ester kann sich dabei von Ergostenolen, Campestenolen, Stigmastenolen, Brassicastenolen sowie vorzugsweise Sitostenolen bzw. Sitostanolen und insbesondere β-Sitostenolen bzw. β-Sitostanolen ableiten. Die Herstellung kann in an sich bekannter Weise erfolgen, beispielsweise durch direkte Veresterung der Stenole mit den Fettsäuren und anschließender Härtung der Ester, durch direkte Veresterung der Stanole mit den Fettsäuren oder vorzugsweise durch Umesterung und gegebenenfalls Härtung der Stenole bzw. Stanole mit den entsprechenden Konjuenfettsäuremethylestern. Ein allgemeines Herstellverfahren durch Umesterung der Stenole/Stanole mit Fettsäureniedrigalkylestern oder Triglyceriden in Gegenwart geeigneter Katalysatoren, wie z.B. Natriumethylat oder speziell auch Enzymen wird in der **EP-A2 0195311** (Yoshikawa) beschrieben. Im Sinne der Erfindung kann die Fettsäurekomponente der Phytostenolester auch in untergeordneten Mengen (kleiner 50 Mol-%) gesättigte, einfach ungesättigte oder mehrfach ungesättigte, nicht-konjugierte Anteile enthalten. Demzufolge kann beispielsweise zur Herstellung der Ester anstelle reiner konjugierter Linolsäure auch eine technische Mischung mit einem hohen Anteil an konjugierter Linolsäure eingesetzt werden, wie sie beispielsweise unter der Bezeichnung Selin® CLA (Grünau) im Handel erhältlich ist. In gleicher Weise können zur Herstellung der Phytostenolester auch entsprechende Fettsäuremethylester oder Triglyceride (z.B. Selin® CLA-TG) mit hohem Konjuengehalt umgeestert werden.

### Tocoaherole

Unter Tocopherolen, die als Potenzierungsmittel für die Phytostenolester in Frage kommen, werden in 2-Stellung mit 4,8,12-Trimethyltridecyl-Resten substituierte Chroman-6-ole (3,4-Dihydro-2-*H*-1-benzopyran-6-ole) verstanden, die der Formel (II) folgen, 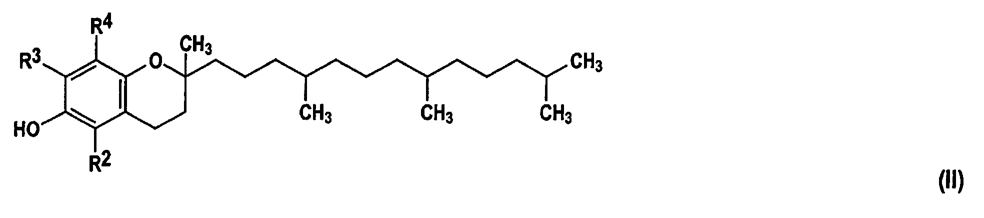 in der R², R³ und R⁴ unabhängig voneinander für Wasserstoff oder eine Methylgruppe stehen. Tocopherole zählen zu den Biochinonen, also polyprenylierten 1,4-Benzo- bzw. Naphthochinonen, deren Prenylketten mehr oder weniger stark gesättigt sind. Typische Beispiele für Tocopherole, die im Sinne der Erfindung als Komponente (b1) in Betracht kommen, sind Ubichinone, Bovichinone, K-Vitamine und/oder Menachinone (2-Methyl-1,4-Naphthochinone). Man unterscheidet bei den Tocopherolen weiterhin α, β, γ-, δ- und ε-Tocopherole, wobei letztere noch über die ursprüngliche ungesättigte Prenylseitenkette verfügen, sowie α-Tocopherolchinon und -hydrochinon, bei denen das Pyran-Ringsystem geöffnet ist. Vorzugsweise wird als Komponente (b) α-Tocopherol (Vitamin E) der Formel (II) eingesetzt, bei der R², R³ und R⁴ für Methylgruppen stehen, oder Ester des α-Tocopherols mit Carbonsäuren mit 2 bis 22 Kohlenstoffatomen, wie beispielsweise α-Tocopherolacetat oder α-Tocopherolpalmitat.

### Chitosane

Chitosane, die ebenfalls als Potenzierungsmittel (b2) für die Phytostenolester in Frage kommen, stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein (III) enthalten: 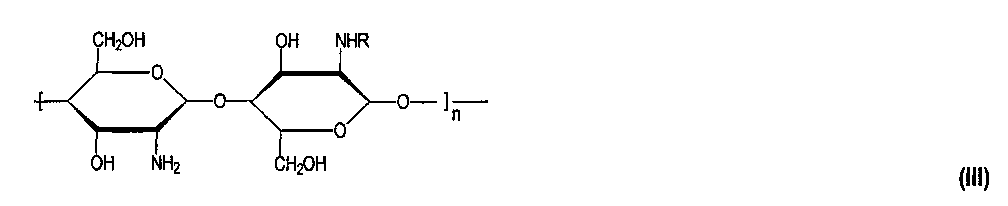 Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. Ullmann's **Encyclopedia of Industrial Chemistry, 5th Ed., Vol. A6, Weinheim, Verlag Chemie, 1986, S. 231-332**). Übersichten zu diesem Thema sind auch beispielsweise von B.Gesslein et al. in **HAPPI 27, 57 (1990),** O.Skaugrud in **Drug Cosm.Ind. 148, 24 (1991)** und E.Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden entweder niedermolekulare Chitosane mit einem durchschnittlichen Molekulargewicht von etwa 50.000 bis etwa 250.000 Dalton oder hochmolekulare Chitosane mit einem durchschnittlichen Molekulargewicht von etwa 500.000 bis etwa 2.000.000 eingesetzt. Entsprechende Verfahren sind beispielsweise aus **Makromol.Chem. 177, 3589 (1976)** oder der französischen Patentanmeldung **FR-A 2701266** bekannt. Besonders bevorzugt werden solche Typen eingesetzt, wie sie in den deutschen Patentanmeldungen **DE-A1 4442987** und **DE-A1 19537001** (Henkel) offenbart werden und die ein durchschnittliches Molekulargewicht von 800.000 bis 1.200.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Neben den Chitosanen als typischen kationischen Biopolymeren kommen im Sinne der Erfindung auch anionisch bzw. nichtionisch derivatisierte Chitosane, wie z.B. Carboxylierungs-, Succinylierungs- oder Alkoxylierungsprodukte in Frage, wie sie beispielsweise in der deutschen Patentschrift **DE-C2 3713099** (L'Oréal) sowie der deutschen Patentanmeldung **DE-A1 19604180** (Henkel) beschrieben werden.

### Phytostenolsulfate

Phytostenolsulfate, die ebenfalls als Potenzierungsmittel (b3) für die Phytostenolester in Frage kommen, stellen bekannte Stoffe dar, die beispielsweise durch Sulfatierung von Phytostenolen mit einem Komplex aus Schwefeltrioxid und Pyridin in Benzol hergestellt werden können **[vgl. J.Am.Chem.Soc. 63, 1259 (1941)].** Typische Beispiele sind die Sulfate von Ergostenolen, Campestenolen, Stigmastenolen und Sitostenolen. Die Phytostenolsulfate können als Alkali- und/oder Erdalkalimetallsalze, als Ammonium-, Alkylammonium-, Alkanolammonium- und/oder Glucammoniumsalze vorliegen. In der Regel werden sie in Form ihrer Natriumsalze eingesetzt.

### (Desoxy)Ribonucleinsäuren

Unter (Desoxy)Ribonucleinsäuren (DNA bzw. RNA), die als letzte Gruppe von Potenzierungsmitteln (b4) für die Phytostenolester in Frage kommen, werden hochmolekulare, fadenförmige Polynucleotide verstanden, die sich von 2'-Desoxy-β-D-ribonucleosiden bzw. D-Ribonucleosiden ableiten, die ihrerseits wieder von äquivalenten Mengen einer Nucleobase und der Pentose 2-Desoxy-D-ribo-furanose bzw. D-Ribofuranose aufgebaut werden. Als Nucleobasen können die DNA bzw. RNA die Purinderivate Adenin und Guanin sowie die Pyrimidine Cytosin und Thymin bzw. Uracil enthalten. In den Nucleinsäuren sind die Nucleobasen N-glykosidisch mit Kohlenstoffatom 1 der Ribose, wodurch im Einzelfall Adenosine, Guanosine, Cytidine und Thimidine entstehen. In den Säuren verknüpft eine Phosphatgruppe die 5'-Hydroxygruppe der Nucleoside mit der 3'-OH-Gruppe der jeweils folgenden durch eine Phospho-diesterbrücke unter Ausbildung von Einzelstrang-DNA bzw. -RNA. Wegen des großen Verhältnisses von Länge zu Durchmesser neigen DNA- bzw. RNA-Moleküle schon bei mechanischer Beanspruchung, etwa bei der Extraktion, zu Strangbruch. Aus diesem Grunde kann das Molekulargewicht der Nucleinsäuren 10³ bis 10⁹ Dalton reichen. Im Sinne der Erfindung werden konzentrierte DNA bzw. RNA-Lösungen eingesetzt, die sich durch ein flüssigkristallines Verhalten auszeichnen. Vorzugsweise werden Desoxy- bzw. Ribonucleinsäuren eingesetzt, die aus marinen Quellen beispielsweise durch Extraktion von Fischsperma erhalten werden und die ein Molekulargewicht im Bereich von 40.000 bis 1.000.000 Dalton aufweisen.

### Gewerbliche Anwendbarkeit

Die Wirkstoffmischungen der Erfindung können die Phytostenolester (a) und die Potenzierungsmittel (b) im Gewichtsverhältnis 99 : 1 bis 1 : 99, vorzugsweise 90 : 10 bis 10 : 90, insbesondere 70 : 25 bis 25 : 75 und besonders bevorzugt 60 : 40 bis 40 : 60 enthalten, wobei allein sicherzustellen ist, daß mit der erfindungsgemäßen Verwendung eine zur Senkung des Cholesteringehaltes im Blut ausreichende Menge der Komponente (a) aufgenommen wird. In einer besonderen Ausführungsform der Erfindung werden die Phytostenolester - alleine oder zusammen mit den Potenzierungsmitteln - in an sich bekannter Weise in Gelatine verkapselt, wobei man die Komponenten (a) und gegebenenfalls (b) jeweils in Mengen von 0,1 bis 50, vorzugsweise 1 bis 30, insbesondere 5 bis 25 und besonders bevorzugt 10 bis 15 Gew.-% - bezogen auf das Gewicht der Gelatinekapseln - einsetzt. Ein weiterer Aspekt der Erfindung betrifft die Erkenntnis, daß die Verkapselung der Phytostenolester in Gelatine eine vorteilhafte Ausführungsform für die orale Aufnahme der Wirkstoffe darstellt.

Eine weitere Verabreichungsform der Phytostenolester sind Zäpfchen, die rektal oder vaginal eingeführt werden können, und als Supositoriengrundmasse ebenfalls Gelatine, gegebenenfalls in Kombination mit Glycerin, oder aber synthetische Fette bzw. Wachse, Polyethylenglycole oder natürliche Bestandteile wie z.B. Kakaobutter enthalten können. Daneben ist es möglich, die Phytostenolester in üblichen Nahrungsmitteln zu lösen bzw. zu dispergieren, als da beispielsweise sind : Salatöle, Dressings, Mayonnaisen, Margarinen, Butter, Fritierfette, Kakaoprodukte, Wurst und dergleichen.

### Beispiele

### Beispiele 1 bis 5, Vergleichsbeispiele V1 bis V3

Es wurden Gelatinekapseln (Gewicht ca. 1,5 g) mit einem Gehalt von 5 Gew.-% verschiedener β-Sitostenolester und gegebenenfalls Vitamin E sowie 0,5 Gew.-% radioaktiv markiertem Cholesterin hergestellt. Zur Untersuchung der hypocholesterinämischen Wirkung ließ man männliche Ratten (Einzelgewicht ca. 200 g) über Nacht fasten. Am folgenden Tag wurde den Versuchstieren jeweils eine zerkleinerte Gelatinekapsel mit etwas kochsalzhaltigem Wasser über eine Magensonde eingeführt. Nach 3, 6, 12, 24 und 48 h wurde den Tieren Blut abgenommen und der Gehalt an radioaktivem Cholesterin bestimmt. Die Ergebnisse, die den Mittelwert der Messungen von 10 Versuchstieren darstellen, sind in Tabelle 1 zusammengefaßt. Die Angaben zur Abnahme der Radioaktivität verstehen sich jeweils in Bezug auf eine Blindgruppe von Versuchstieren, denen lediglich Gelatinekapseln mit einem Gehalt von 20 Gew.-% Vitamin E und einer entsprechenden Menge radioaktiv markiertem Cholesterin verabreicht worden war. Die Mischungen 1 bis 5 sind erfindungsgemäß, die Mischungen V1 bis V3 dienen dem Vergleich.

**Tabelle 1**

| **Hypocholesterinämische Wirkung (Mengenangaben als Gew.-% bezogen auf Gelatinekapsel)** | | | | | | | | |
|---|---|---|---|---|---|---|---|---|
| **Zusammensetzung/Aktivität** | **1** | **2** | **3** | **4** | **5** | **V1** | **V2** | **V3** |
| Konjuenfettsäure-β-sitostenolester* | 5 | - | - | - | - | - | - | - |
| konj. C₁₂-C₂₄-Fischfettsäure-β-sitostenolester | - | 5 | - | - | - | - | - | - |
| Konjuenfettsäure-β-sitostanolester* | - | - | 5 | - | - | - | - | - |
| konj. C₁₂-C₂₄-Fischfettsäure-β-sitostenolester | - | - | - | 5 | 5 | - | - | - |
| Laurinsäure-β-sitostanolester | - | - | - | - | - | - | - | - |
| Ölsäure-β-sitostanolester | - | - | - | - | - | 5 | - | - |
| Linolsäure-β-sitostanolester | - | - | - | - | - | - | 5 | - |
| Vitamin E | - | - | - | - | 5 | - | - | 5 |

| ***Radioaktivität [%-rel]*** | | | | | | | | |
|---|---|---|---|---|---|---|---|---|
| ***- nach 3 h*** | 95 | 95 | 95 | 95 | 95 | 95 | 95 | 95 |
| ***- nach 6 h*** | 80 | 79 | 78 | 78 | 75 | 84 | 82 | 83 |
| ***- nach 12 h*** | 72 | 70 | 68 | 67 | 61 | 76 | 74 | 73 |
| ***- nach 24 h*** | 45 | 45 | 43 | 43 | 39 | 51 | 48 | 47 |
| ***- nach 48 h*** | 21 | 20 | 18 | 17 | 15 | 30 | 26 | 25 |

| | | | | | | | | |
|---|---|---|---|---|---|---|---|---|
| *) Fettsäurebasis: Selin® CLA (Grünau/Illertissen) | | | | | | | | |

## Patentansprüche

1. Verwendung von Estern von Phytostenolen mit konjugierter Linolsäure und/oder konjugierter Fischfettsäure zur Herstellung von Mitteln zur Verminderung des Cholesterin gehaltes im serum.

2. Verwendung nach Anspruch 1, **dadurch gekennzeichnet, daß** man Ester von β-Sitostenol oder β-Sitostanol einsetzt.

3. Verwendung nach den Ansprüchen 1 und/oder 2, **dadurch gekennzeichnet, daß** man die Phytostenolester zusammen mit Potenzierungsmitteln einsetzt, die ausgewählt sind aus der Gruppe, die gebildet wird von Tocopherolen, Chitosanen, Phytostenolestern und (Desoxy)-Ribonucleinsäuren sowie deren Gemischen.

4. Verwendung nach den Ansprüchen 1 bis 3, **dadurch gekennzeichnet, daß** man als Potenzierungsmittel Vitamin E einsetzt.

5. Verwendung nach den Ansprüchen 1 bis 4, **dadurch gekennzeichnet, daß** man als Potenzierungsmittel Chitosane mit einem durchschnittlichen Molekulargewicht im Bereich von 50.000 bis 250.000 bzw. 500.000 bis 2.000.000 Dalton einsetzt.

6. Verwendung nach den Ansprüchen 1 bis 5, **dadurch gekennzeichnet, daß** man als Potenzierungsmittel) marine Desoxyribonucleinsäuren einsetzt, die ein Molekulargewicht im Bereich von 40.000 bis 1.000.000 Dalton aufweisen.

7. Verwendung nach den Ansprüchen 1 bis 6, **dadurch gekennzeichnet, daß** man die Komponenten (a) und gegebenenfalls (b) in Gelatine verkapselt.

8. Verwendung nach Anspruch 7, **dadurch gekennzeichnet, daß** man die Phytostenolester in Mengen von 0,1 bis 50 Gew.-% - bezogen auf das Gewicht der Gelatinekapseln - einsetzt.

## Claims

1. The use of esters of phytostenols with conjugated linoleic acid and/or conjugated fish fatty acid for the production of preparations for reducing the cholesterol content in serum.

2. The use claimed in claim 1, **characterized in that** esters of β-sitostenol or β-sitostanol are used.

3. The use claimed in claims 1 and/or 2, **characterized in that** the phytostenol esters are used together with potentiating agents selected from the group consisting of tocopherols, chitosans, phytostenol esters and (deoxy)ribonucleic acids and mixtures thereof.

4. The use claimed in claims 1 to 3, **characterized in that** vitamin E is used as the potentiating agent.

5. The use claimed in claims 1 to 4, **characterized in that** chitosans with an average molecular weight of 50,000 to 250,000 or 500,000 to 2,000,000 dalton are used as potentiating agents.

6. The use claimed in claims 1 to 5, **characterized in that** marine deoxyribonucleic acids with a molecular weight of 40,000 to 1,000,000 dalton are used as potentiating agents.

7. The use claimed in claims 1 to 6, **characterized in that** components (a) and optionally (b) are encapsulated in gelatin.

8. The use claimed in claim 7, **characterized in that** the phytostenol esters are used in quantities of 0.1 to 50% by weight, based on the weight of the gelatin capsules.

## Revendications

1. Utilisation d'esters de phytosténols avec de l'acide linoléique conjugué et/ou des acides gras de poisson conjugués, en vue de la production d'agents destinés à la diminution de la teneur en cholestérol dans le sérum.

2. Utilisation selon la revendication 1,
**caractérisée en ce qu'**
on met en oeuvre des esters de β-sitosténol ou de β-sitostanol.

3. Utilisation selon les revendications 1 et/ou 2,
**caractérisée en ce qu'**
on met en oeuvre les esters de phytosténol conjointement avec des agents potentialisateurs choisis dans le groupe formé des tocophérols, des chitosanes, des esters de phytosténols et des acides (désoxy)ribonucléique ainsi que de leurs mélanges.

4. Utilisation selon les revendications 1 à 3,
**caractérisée en ce qu'**
on utilise comme agent potentialisateur de la vitamine E.

5. Utilisation selon les revendications 1 à 4,
**caractérisée en ce qu'**
on utilise comme agent potentialisateur des chitosanes ayant un poids moléculaire moyen dans la zone de 50 000 à 250 000 ou de 500 000 à 2 000 000 daltons.

6. Utilisation selon les revendications 1 à 5,
**caractérisée en ce qu'**
on utilise comme agent potentialisateur des acides désoxyribonucléiques marins qui possèdent un poids moléculaire dans la zone de 40 000 à 1 000 000 daltons.

7. Utilisation selon les revendications 1 à 6,
**caractérisée en ce qu'**
on encapsule les composants a) et éventuellement b) dans de la gélatine.

8. Utilisation selon la revendication 7,
**caractérisée en ce qu'**
on utilise les esters de phytosténol en quantités s'échelonnant de 0,1 à 50 % en poids - rapporté au poids des capsules de gélatine.
